# EUROPEAN PATENT APPLICATION

(11) **EP 4 056 264 A1**
(43) Date of publication of application: **14.09.2022**
(21) Application number: 22160420.0
(22) Date of filing: 07.03.2022
(51) Int. Cl.: B01J 20/04, B01J 20/14, B01J 20/24, B01J 20/26, C07C 401/00

(54) **SOLID PHASE MIXTURE COMPRISING AN OXIDATING AGENT OR A SALT THEREOF, PACKING MATERIAL, AND COLUMN**

(30) Priority: 09.03.2021 JP 2021037510
(71) Applicant: Jeol Ltd., Akishima, Tokyo 196-8558 (JP)
(72) Inventor: FUKUZAWA, Seketsu, Akishima, 1968558 (JP); TAKAHASHI, Koji, Akishima, 1968558 (JP)
(74) Representative: Boult Wade Tennant LLP

(57) **Abstract**

[Problems] To provide a solid phase mixture that allows for further speedup, further simplification, and further cost reduction in the detection and quantification of vitamin D metabolites (particularly 1α,25-dihydroxyvitamin D₃) in living organisms, a packing material containing the solid phase mixture, and a column packed with the packing material.

[Solution] A solid phase mixture including an oxidizing agent and/or a salt of the oxidizing agent and solid phase particles, wherein the oxidizing agent is a compound capable of selectively oxidizing 1,2-diol compounds, is provided. In addition, a packing material containing the solid phase mixture is provided. Further, a column packed with the packing material is provided.

## Description

### Technical Field

The present invention relates to a solid phase mixture, a packing material, and a column.

### Background Art

The detection/quantification of vitamin D metabolites in living organisms is in great demand in the fields of clinical examination, food nutrition, and public health. Among them, the most important metabolite having a bioactive effect is 1α,25-dihydroxyvitamin D₃, which is called "active vitamin D", and its selective extraction is extremely important.

For example, Non-Patent Literature 1 proposes a technique related to the immunoextraction of 1α,25-dihydroxyvitamin D₃ using an anti-1α,25-dihydroxyvitamin D antibody column. In addition, Non-Patent Literature 2 proposes a technique related to the periodic acid treatment of plasma extracts.

### Citation List

### Non-Patent Literature

Non-Patent Literature 1: Improved sensitivity of serum/plasma 1α,25-dihydroxyvitamin D quantification by DAPTAD derivatization., Ishige, T.; Satoh, M.; Ogawa, S.; Nishimura, N.; Matsushita, K.; Higashi, T.; Nomura, F., Clin. Chem. Acta 2017, 473, 173-179.
Non-Patent Literature 2: Simultaneous measurement of plasma vitamin D3 metabolites, including 4β,25-dihydroxyvitamin D3, using liquid chromatography-tandem mass spectrometry., Wang, Z.; Senn, T.; Kalhom, T.; Zheng, X. E.; Zheng, S.; Davis, C. L.; Hebert, M. F.; Lin, Y. S.; Thummel, K. E., Anal. Biochem. 2011, 418, 126-133.

### Summary of Invention

### Technical Problem

However, with the techniques proposed in Non-Patent Literatures 1 and 2, in the detection and quantification of vitamin D metabolites (particularly 1α,25-dihydroxyvitamin D₃) in living organisms, the achievement of further speedup, the achievement of further simplification, and the achievement of further cost reduction may be impossible.

Thus, the present invention has been made in light of such circumstances, and a main object thereof is to provide a solid phase mixture that allows for further speedup, further simplification, and further cost reduction in the detection and quantification of vitamin D metabolites (particularly 1α,25-dihydroxyvitamin D₃) in living organisms, a packing material containing the solid phase mixture, and a column packed with the packing material.

### Solution to Problem

The present inventors have conducted extensive research to achieve the above main object, and as a result, surprisingly, successfully developed a solid phase mixture that allows for further speedup, further simplification, and further cost reduction in the detection and quantification of vitamin D metabolites (particularly 1α,25-dihydroxyvitamin D₃) in living organisms, a packing material containing the solid phase mixture, and a column packed with the packing material. The present invention has thus been accomplished.

That is, the present invention provides, as a first aspect,
a solid phase mixture including: an oxidizing agent and/or a salt of the oxidizing agent; and solid phase particles.

In the first aspect according to the present invention, it is also possible that
the oxidizing agent is a compound capable of selectively oxidizing 1,2-diol compounds.

In the first aspect according to the present invention, it is also possible that
the oxidizing agent is periodic acid or lead tetraacetate.

In the first aspect according to the present invention, it is also possible that
the salt of the oxidizing agent is a salt of periodic acid.

In the first aspect according to the present invention, it is also possible that
the solid phase particles are particles of at least one kind selected from diatomaceous earth, silica gel, dextran, and a polymer.

In the first aspect according to the present invention, it is also possible that
the polymer is at least one kind selected from polystyrene, polymethacrylate, and polyvinyl alcohol.

In the first aspect according to the present invention, it is also possible that
the mass of the oxidizing agent and/or the salt of the oxidizing agent relative to the mass of the solid phase particles is 4% to 20%.

In the first aspect according to the present invention, it is also possible that
the solid phase mixture is configured to selectively bind to and oxidize a 1,2-diol compound, thereby extracting a diol compound other than 1,2-diol compounds.

In the first aspect according to the present invention, it is also possible that
the 1,2-diol compound is a vitamin D having a 1,2-diol structure, and
the diol compound other than 1,2-diol compounds is a vitamin D having a diol structure other than a 1,2-diol structure.

In the first aspect according to the present invention, it is also possible that
the solid phase mixture is configured to selectively bind to and oxidize a 1,2-diol compound, thereby extracting a 1,3-diol compound.

In the first aspect according to the present invention, it is also possible that
the 1,2-diol compound is a vitamin D having a 1,2-diol structure, and
the 1,3-diol compound is a vitamin D having a 1,3-diol structure.

In the first aspect according to the present invention, it is also possible that
the oxidizing agent and/or the salt of the oxidizing agent is in a solid phase state.

In addition, the present invention provides, as a second aspect,
a packing material including the solid phase mixture of the first aspect according to the present invention.

Further, the present invention provides, as a third aspect,
a column packed with the packing material of the second aspect according to the present invention.

### Advantageous Effects of Invention

According to the present invention, in the detection and quantification of vitamin D metabolites (particularly 1α,25-dihydroxyvitamin D₃) in living organisms, further speedup, further simplification, and further cost reduction can be achieved. Incidentally, the effects described here are not necessarily limited, and any of the effects described herein may be applied.

### Brief Description of Drawings

[Fig. 1] Fig. 1 shows SRM chromatograms of 1α,25-dihydroxyvitamin D₃ (1α,25(OH)₂D₃) and 4β,25-dihydroxyvitamin D₃ (4β,25(OH)₂D₃).
[Fig. 2] Fig. 2 shows SRM chromatograms about 4β,25(OH)₂D₃ being able to be removed by the addition of periodic acid.
[Fig. 3] Fig. 3 shows SRM chromatograms about 4β,25(OH)₂D₃ being able to be removed by the addition of periodic acid.
[Fig. 4] Fig. 4 shows SRM chromatograms about 4β,25(OH)₂D₃ being able to be removed by the addition of periodic acid in pooled serum.

### Description of Embodiments

Hereinafter, preferred modes for carrying out the present invention will be described. The embodiments described below show some examples of typical embodiments of the present invention, and do not narrow the interpretation of the scope of the present invention.

Incidentally, the description will be given in the following order.
1. Outline of the Present Invention
2. First Embodiment (Example of Solid Phase Mixture)
3. Second Embodiment (Example of Packing Material)
4. Third Embodiment (Example of Column)
5. Examples
   5-1. Example 1
   5-2. Example 2
   5-3. Example 3

### <1. Outline of the Present Invention>

First, the outline of the present invention will be described. The present invention relates to a solid phase mixture, a packing material containing the solid phase mixture, and a column packed with the packing material.

LC-MS/MS is a combination of chromatographic separation and the m/z (mass-to-charge ratio) of fragment ions and has high separation ability. 24R,25-dihydroxyvitamin D₃ can be separated by the m/z of fragment ions, whereas 4β,25-dihydroxyvitamin D₃ cannot be separated by the m/z of fragment ions.

The reaction formula of the derivatization reaction of 1α,25-dihydroxyvitamin D₃ (1α,25(OH)₂D₃), 24R,25-dihydroxyvitamin D₃ (24R,25(OH)₂D₃), and 4β,25-dihydroxyvitamin D₃ (4β,25(OH)₂D₃) by a derivatization reagent DAP-PA (1) is as follows. As a result of the derivatization reaction, a 1α,25(OH)₂D₃-DAPTAD derivative (7), a 24R,25(OH)₂D₃-DAPTAD derivative (8), and a 4β,25(OH)₂D₃-DAPTAD derivative (9) are formed, and, using these three derivatives, LC-MS/MS analysis is performed.

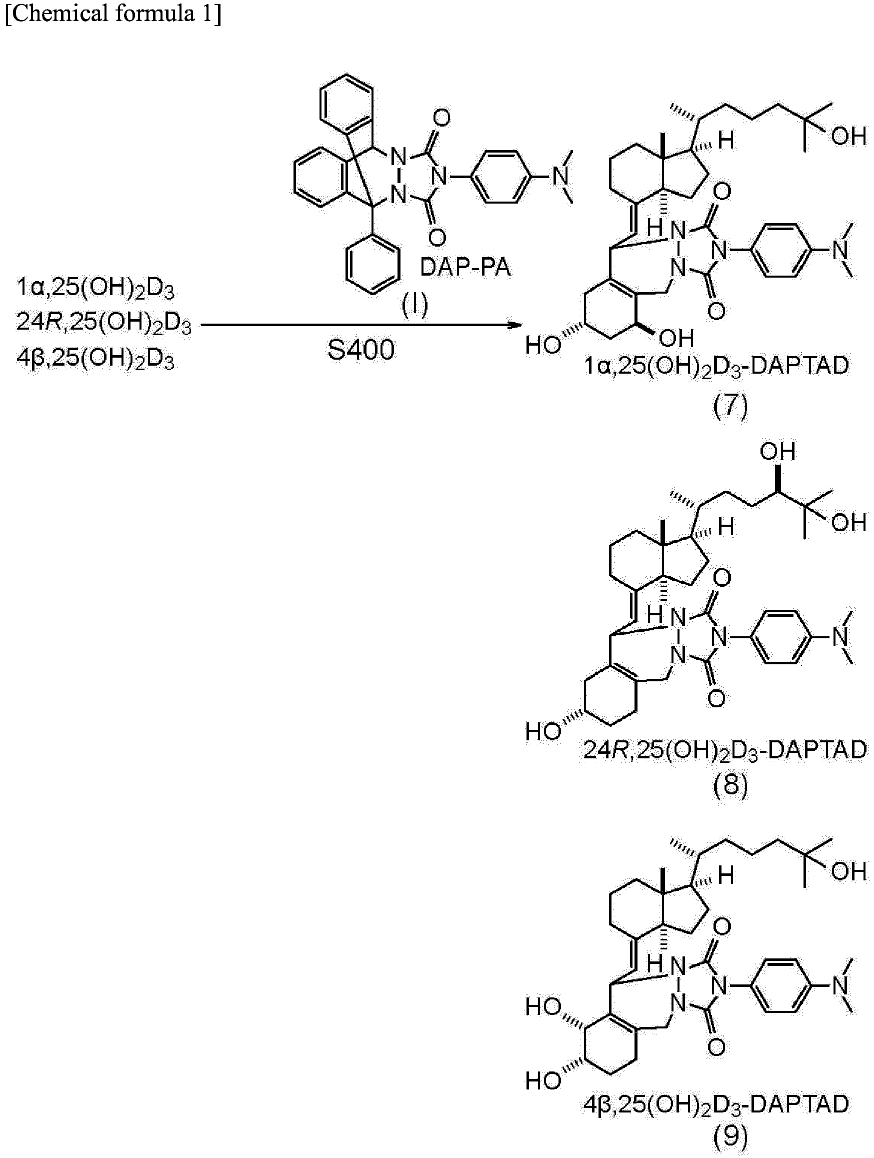

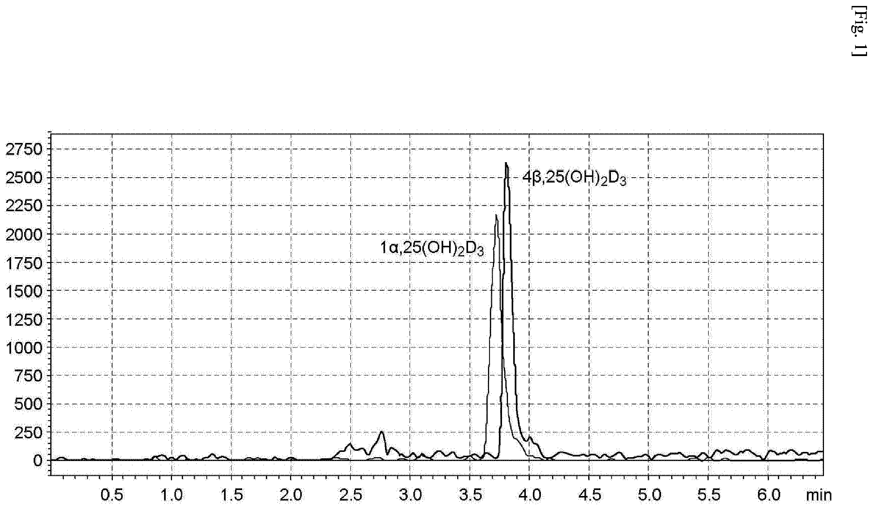

Description will be given with reference to Fig. 1. Fig. 1 shows SRM chromatograms of 1α,25-dihydroxyvitamin D₃ (1α,25(OH)₂D₃) and 4β,25-dihydroxyvitamin D₃ (4β,25(OH)₂D₃). Incidentally, 1α,25-dihydroxyvitamin D₃ (1α,25(OH)₂D₃) is a ¹³C₃-labeled form. The analysis conditions are shown in Table 1 below.

**[Table 1]**

| Analysis Conditions | | | | | |
|---|---|---|---|---|---|
| | LC | Shimadzu UFLC LC-20AD | | | |
| | Column | CAPCELL CORE C₁₈ (2.7 µm) 2.1 mm I.D. x 100 mm column (OSAKA SODA, Osaka, Japan) | | | |
| | Solvent | A: 0.1% Formic acid-water | | | |
| | | B: 0.1% Formic acid-acetonitrile | | | |

| | Flow rate | | 0.2 mL/min | | |
|---|---|---|---|---|---|
| | 0 min | 30%B | | | |
| | 0.5 | 60%B | | | |
| | 3.5 | 60%B | | | |
| | 3.51 | 90%B | | | |
| | 5 | 90%B | | | |
| | 5.01 | 30%B | | | |
| | 6.5 | 30%B | | | |
| | | | | | |

| | MS | | Shimadzu LCMS8040 | | |
|---|---|---|---|---|---|
| | MS/MS conditions | | *m*/*z* | CE | |
| | 1α,25(OH)₂D₃ | | 635.3 > 357.0 | 25 | |
| | 4β,25(OPH)₂D₃ | | | | |
| | 1α,25(OH)₂D₃-¹³C₃ | | 638.3 > 357.0 | 25 | |

As is clear from Fig. 1, chromatographic separation between 1α,25-dihydroxyvitamin D₃ and 4β,25-dihydroxyvitamin D₃ is difficult.

Therefore, under the current circumstances, their isolation and extraction is possible only through immunoextraction using an anti-1α,25-dihydroxyvitamin D antibody column.

Next, conventional Technical Example 1 (immunoextraction of 1α,25-dihydroxyvitamin D₃ using an anti-1α,25-dihydroxyvitamin D antibody column) and Technical Example 2 (periodic acid treatment of plasma extracts) will be described. The conventional Technical Example 1 is as follows.

The buffer in the antibody column is removed by centrifugation. 0.3 mL of a phosphate buffer containing 0.2 mL of serum with an internal standard is loaded onto the column and, while being slowly rotated, incubated for 1 hour. The sample diluent is removed by centrifugation, and then a wash solution is added and removed by centrifugation (repeat twice). An eluate is added, followed by centrifugal elution and recovery. The eluate is concentrated, and then a derivatization reagent DAPTAD or PTAD (0.2 mg/mL, 50 µL) is added and allowed to stand at room temperature for 1 hour to cause derivatization. After concentration, the residue is dissolved with 33 µL of a redissolution solvent (30% acetonitrile solution) and subjected to LC-MS/MS analysis.

The scheme of Technical Example 1 is shown below as Scheme 1.

### (Scheme 1)

(1) Remove the buffer in the antibody column by centrifugation.
   ↓
(2) Load 0.5 mL of a sample diluent containing 0.2 mL of serum onto the column.
   ↓
(3) Incubate for 1 hour while slowly rotating.
   ↓
(4) Remove the sample diluent by centrifugation.
   ↓
(5) Add a wash solution and remove by centrifugation (repeat twice).
   ↓
(6) Add an eluate and elute by centrifugation.
   ↓
(7) Add a derivatization reagent DAPTAD or PTAD (0.2 mg/mL, 50 µL) and allow to stand at room temperature for 1 hour to cause derivatization.
   ↓
(8) Dissolve with 33 µL of a redissolution solvent (30% acetonitrile solution).
   ↓
(9) LC-MS/MS analysis.

The conventional Technical Example 2 is as follows.

A sample after derivatization is dissolved in 0.3 mL of a redissolution solvent (50% methanol solution). 30 µL of a 5% aqueous sodium periodate solution is added and incubated for 1 hour. 0.5 mL of water is added and extracted with 2 mL of ethyl acetate. After concentration, the residue is dissolved with 40 µL of a redissolution solvent (40% acetonitrile solution) and subjected to LC-MS/MS analysis.

The scheme of Technical Example 2 is shown below as Scheme 2.

### (Scheme 2)

(1) Dissolve a sample after derivatization in 0.3 mL of a redissolution solvent (50% methanol solution).
   ↓
(2) Add 30 µL of a 5% aqueous sodium periodate solution and incubate for 1 hour.
   ↓
(3) Add 0.5 mL of water and extract with 2 mL of ethyl acetate.
   ↓
(4) After concentration, dissolve with 40 µL of a redissolution solvent (40% acetonitrile solution).
   ↓
(5) LC-MS/MS analysis.

Technical Example 1 is disadvantages in that expensive antibodies are used, and, because incubation for 1 hour is necessary, the treatment takes a long time. In contrast to the conventional method for selectively extracting 1α,25-dihydroxyvitamin D₃ using an antigen-antibody reaction, in the present invention, focusing on the fact that 4β,25-dihydroxyvitamin D₃, which is a contaminant in biological samples, has a 1,2-diol structure, compounds having a 1,2-diol structure (e.g., 4β,25-dihydroxyvitamin D₃) are removed by their selective reaction/decomposition using periodic acid, and, as a result, 1α,25-dihydroxyvitamin D₃ is selectively extracted.

In addition, in Technical Example 2, although 4β,25-dihydroxyvitamin D₃ is decomposed by periodic acid, it takes time and effort as after vitamin Ds in plasma are deproteinized and then derivatized, an aqueous sodium periodate solution is added and incubated for 1 hour, followed by ethyl acetate extraction. According to the present invention, production is possible in a simple manner, for example, by simply adding sodium periodate in solid form to a column (it is preferable not use an aqueous sodium periodate solution), and, as in the ordinary deproteinization treatment using a column, 4β,25-dihydroxyvitamin D₃ can be removed by simply passing through the column; thus, the invention achieves quick, simple, and inexpensive extraction of 1α,25-dihydroxyvitamin D₃.

The present invention is, for example, an extraction column available for the accurate detection of 1α,25-dihydroxyvitamin D₃ using mass spectrometry, and considers LC-MS/MS (Liquid Chromatography-Tandem Mass Spectrometer) as its detection means. Then, the present invention is used without limitation to mass spectrometer manufacturers. The demand for the measurement of 1α,25-dihydroxyvitamin D₃ in the field of clinical examination is huge. Compared to the immunoextraction method using an antibody, the present invention allows for cost reduction, a remarkable decrease in the treatment time, and a dramatic increase in the examination speed.

Hereinafter, preferred modes for carrying out the present invention will be described in detail. The embodiments described below show some examples of typical embodiments of the present invention, and do not narrow the interpretation of the scope of the present invention.

### <2. First Embodiment (Example of Solid Phase Mixture)>

A solid phase mixture of a first embodiment (Example of Solid Phase Mixture) according to the present invention is a solid phase mixture including: an oxidizing agent and/or a salt of the oxidizing agent; and solid phase particles.

The oxidizing agent is a compound capable of selectively oxidizing 1,2-diol compounds. The oxidizing agent is not particularly limited, but is preferably periodic acid or lead tetraacetate. The salt of the oxidizing agent is not particularly limited either, but is preferably a salt of periodic acid. It is preferable that the oxidizing agent and/or the salt of the oxidizing agent is in a solid phase state.

The solid phase particles may have a hydrophilic functional group, and, for example, particles of at least one kind selected from diatomaceous earth, silica gel, dextran, and a hydroxylated synthetic polymer can be mentioned. As the hydroxylated synthetic polymer, for example, at least one kind selected from polystyrene, polymethacrylate, and polyvinyl alcohol can be mentioned.

In the solid phase mixture of the first embodiment according to the present invention, the mass of the oxidizing agent and/or the salt of the oxidizing agent relative to the mass of the solid phase particles may be any ratio, but is preferably 4% to 20%.

The oxidizing agent selectively binds to and oxidizes a 1,2-diol compound to remove the 1,2-diol compound, thereby extracting a compound having no 1,2-diol structure (i.e., diol compound other than 1,2-diol). As the diol compound other than 1,2-diol compounds, for example, a 1,3-diol compound can be mentioned.

As in the following reaction formula, in Step S100, the 1,2-diol compound (1) reacts with sodium periodate/water (NaIO₄/H₂O) to form a complex compound (3) of a 1,2-diol compound and periodic acid, but the 1,3-diol compound (2) does not react with sodium periodate/water (NaIO₄/H₂O).

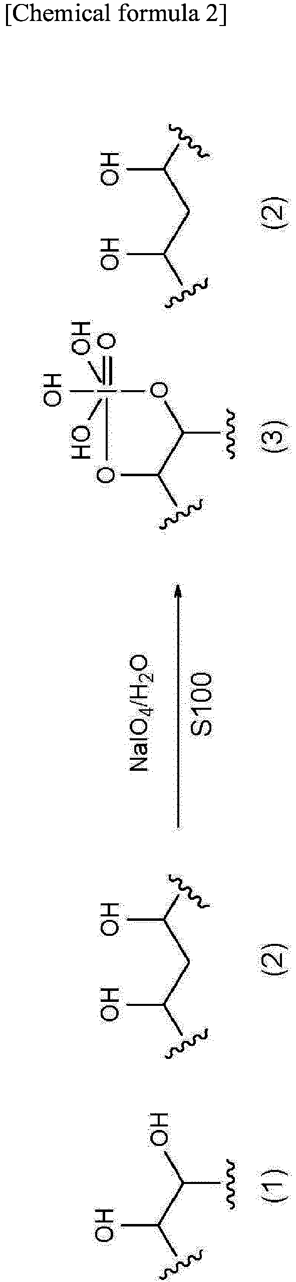

The solid phase mixture of the first embodiment according to the present invention selectively binds to and oxidizes, among vitamin Ds, those having a 1,2-diol structure (4β,25-dihydroxyvitamin D₃ (5), 24R,25-dihydroxyvitamin D₃ (6), etc.). As a result, a complex compound (5-1) of 4β,25-dihydroxyvitamin D₃ and periodic acid is formed, and a complex compound (6-1) of 24R,25-dihydroxyvitamin D₃ and periodic acid is formed. Vitamin Ds having no 1,2-diol structure can be extracted.

A typical example of a compound having no 1,2-diol structure is 1α,25-dihydroxyvitamin D₃ (4) (or (7)) having a 1,3-diol structure. In addition, la,25-dihydroxyvitamin D₂ (8), (23S,25R)-1α,25-dihydroxyvitamin D₃ 26,23-lactone (9), and the like can also be extracted as with 1α,25-dihydroxyvitamin D₃ (4) (and (7)).

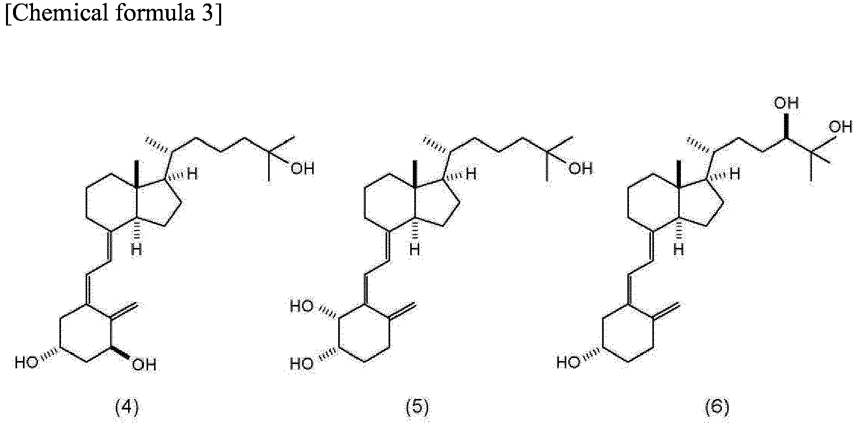

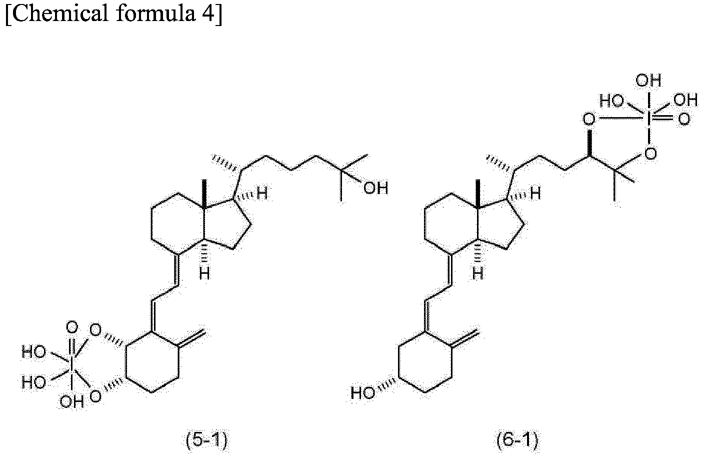

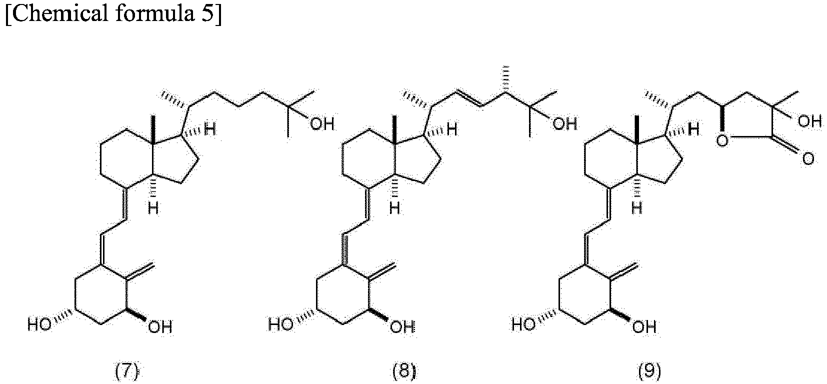

As a mode of use of the solid phase mixture of the first embodiment according to the present invention, for example, use in the following manner is possible: the solid phase mixture is added to a packing material of the below-described second embodiment according to the present invention, and such a packing material is packed into a column of the below-described third embodiment according to the present invention (column method). In addition, as a mode of use of the solid phase mixture of the first embodiment according to the present invention, for example, it is possible that the solid phase mixture itself is mixed with the solution to be subjected to extraction (batch method).

The solid phase mixture according to the present invention selectively removes dihydroxyvitamin D₃s having a 1,2-diol structure from biological samples (blood, serum, plasma, urine, saliva, spinal fluid, etc.) by the column method or batch method, and extracts 1α,25-dihydroxyvitamin D₃s having no 1,2-diol structure, etc. The extracted 1α,25-dihydroxyvitamin D₃s are detected and quantified by the LC-MS/MS method.

### <3. Second Embodiment (Example of Packing Material)>

A packing material of a second embodiment (Example of Packing Material) according to the present invention is a packing material containing a solid phase mixture according to the present invention (e.g., solid phase mixture of the first embodiment according to the present invention).

### <4. Third Embodiment (Example of Column)>

A column of a third embodiment (Example of Column) according to the present invention is a column packed with a packing material according to the present invention (e.g., packing material of the second embodiment according to the present invention).

### <5. Examples>

Hereinafter, the effects and the like of the present invention will be described in detail with reference to examples. Incidentally, the scope of the present invention is not limited to the examples.

### <5-1. Example 1>

### [Production of Diatomaceous Earth Column with Periodic Acid Added]

Each amount of sodium periodate (0 g, 0.04 g, 0.06 g, 0.1 g, 0.15 g, or 0.2 g) was added to 1 g of diatomaceous earth and mechanically mixed well to prepare five solid phase mixtures. The five solid phase mixtures were each packed into a 3-mL syringe type column.

### <5-2. Example 2>

### [Removal of 4β,25-Dihydroxyvitamin D₃ Using Periodic Acid-Added Column (0.15 g of sodium periodate is added to 1 g of diatomaceous earth)]

1 mL of a diluent (30% acetonitrile solution) containing both a 4β,25-dihydroxyvitamin D₃ standard (1.8 pg, 18 pg, 180 pg, or 1,800 pg) and an internal standard (1α,25-dihydroxyvitamin D₃-¹³C₃) was loaded onto the column. The column was allowed to stand for 1 minute, and 0.6 mL of hexane was added, followed by elution under pressure. The eluate was discarded. 0.6 mL of hexane/ethyl acetate (1:1) was added, followed by elution under pressure (repeated twice). The eluate was concentrated, and then a derivatization reagent DAP-PA (0.5 mg/mL, 0.1mL) was added and heated at 80°C for 15 minutes to cause derivatization. After concentration, the residue was dissolved with 50 µL of redissolution solvent (50% acetonitrile solution) and subjected to LC-MS/MS analysis.

The scheme of Example 2 is shown below as Scheme 3.

### (Scheme 3)

(1) Load 1 mL of a sample diluent (30% acetonitrile solution) containing both a 4β,25-dihydroxyvitamin D₃ standard (1.8 pg, 18 pg, 180 pg, or 1,800 pg) and an internal standard (1α,25-dihydroxyvitamin D₃-¹³C₃) onto the column.
   ↓
(2) Allow to stand for 1 minute.
   ↓
(3) Add 0.6 mL of hexane and elute under pressure. Discard the eluate.
   ↓
Add 0.6 mL of hexane/ethyl acetate (1:1) and elute under pressure (repeat twice).
   ↓
(4) Concentrate the eluate, add a derivatization reagent DAP-PA (0.5 mg/mL, 0.1mL), and heat at 80°C for 15 minutes to cause derivatization.
   ↓
(5) After concentration, dissolve with 50 µL of a redissolution solvent (50% acetonitrile solution).
   ↓
(6) LC-MS/MS analysis.

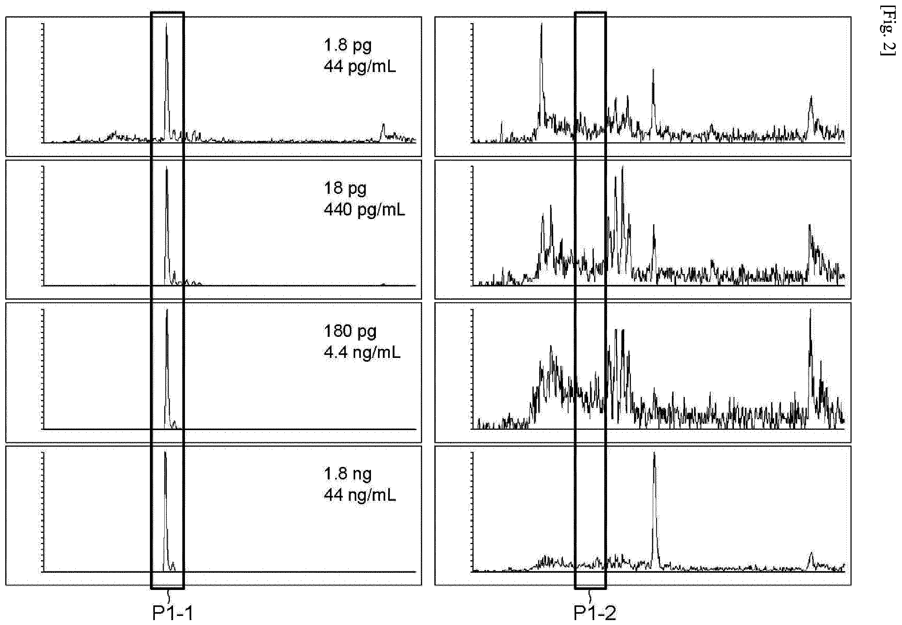

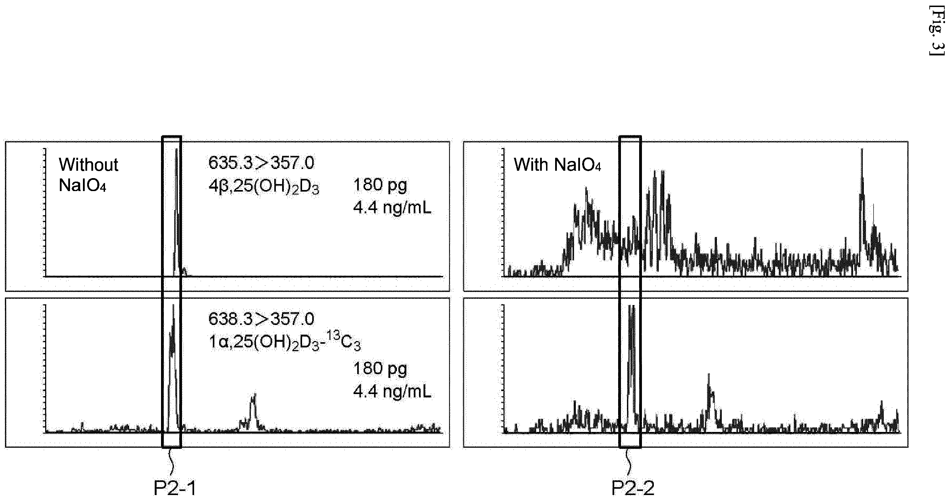

The results of the LC-MS/MS analysis of Example 2 are shown in Figs. 2 and 3. The conditions for the LC-MS/MS analysis for deriving the results of the LC-MS/MS analysis shown in Fig. 2 are shown below in Table 2. In addition, the conditions for the LC-MS/MS analysis for deriving the results of the LC-MS/MS analysis shown in Fig. 3 are shown below in Table 3.

**[Table 2]**

| Analysis Conditions | | | | | |
|---|---|---|---|---|---|
| | LC | Agilent Technologies model 1290 Infinity | | | |
| | Column | CAPCELL CORE C₁₈ (2.7 µm) 2.1 mm I.D. × 100 mm column (OSAKA SODA, Osaka, Japan) | | | |
| | Solvent | A: 0.1% Formic acid-water | | | |
| | | B: 0.1% Formic acid-acetonitrile | | | |

| | Flow rate | | 0.3 mL/min | | |
|---|---|---|---|---|---|
| | 0.01 min | 30%B | | | |
| | 0.5 | 58%B | | | |
| | 7.0 | 58%B | | | |
| | 7.01 | 90%B | | | |
| | 8.0 | 90%B | | | |
| | 8.01 | 30%B | | | |
| | 9.0 | 30%B | | | |
| | | | | | |

| | MS | | SCIEX QTRAP 4500 | | |
|---|---|---|---|---|---|
| | MS/MS conditions | | *m*/*z* | CE | |
| | 1α,25(OH)₂D₃ | | 635.3 > 357.0 | 25 | |
| | 4β,25(OH)₂D₃ | | | | |
| | 1α,25(OH)₂D₃-¹³C₃ | | 638.3 > 357.0 | 25 | |

**[Table 3]**

| Analysis Conditions | | | | | |
|---|---|---|---|---|---|
| | LC | Agilent Technologies model 1290 Infinity | | | |
| | Column | CAPCELL CORE C₁₈ (2.7 µm) 2.1 mm I.D. × 100 mm column (OSAKA SODA, Osaka, Japan) | | | |
| | Solvent | A: 0.1% Formic acid-water | | | |
| | | B: 0.1% Formic acid-acetonitrile | | | |

| | Flow rate | | 0.3 mL/min | | |
|---|---|---|---|---|---|
| | 0.01 min | 30%B | | | |
| | 0.5 | 58%B | | | |
| | 7.0 | 58%B | | | |
| | 7.01 | 90%B | | | |
| | 8.0 | 90%B | | | |
| | 8.01 | 30%B | | | |
| | 9.0 | 30%B | | | |
| | | | | | |

| | MS | | SCIEX QTRAP 4500 | | |
|---|---|---|---|---|---|
| | MS/MS conditions | | *m*/*z* | CE | |
| | 1α,25(OH)₂D₃ | | 635.3 > 357.0 | 25 | |
| | 4β,25(OPH)₂D₃ | | | | |
| | 1α,25(OH)₂D₃-¹³C₃ | | 638.3 > 357.0 | 25 | |

Fig. 2 shows SRM chromatograms about 4β,25(OH)₂D₃ being able to be removed by the addition of periodic acid (sodium periodate).

More specifically, the left diagrams in Fig. 2 show the SRM chromatograms for columns with diatomaceous earth alone. Then, the left diagrams in Fig. 2 show, from upper to lower, the SRM chromatograms with the addition of 4β,25-dihydroxyvitamin D₃ in amounts of 1.8 pg (44 pg/mL), 18 pg (440 pg/mL), 180 pg (4.4 ng/mL), and 1.8 ng (44 ng/mL). In addition, the right diagrams in Fig. 2 show the SRM chromatograms for columns with 0.15 g of sodium periodate added per 1 g of diatomaceous earth. Then, the right diagrams in Fig. 2 show, from upper to lower, the SRM chromatograms with the addition of 4β,25-dihydroxyvitamin D₃ in amounts of 1.8 pg (44 pg/mL), 18 pg (440 pg/mL), 180 pg (4.4 ng/mL), and 1.8 ng (44 ng/mL).

As is clear with reference to the region indicated by the reference numeral P1-1 in the left diagrams in Fig. 2, it was confirmed that when periodic acid (sodium periodate) was not added, 4β,25(OH)₂D₃ was not removed but extracted. Meanwhile, as is clear with reference to the region indicated by the reference numeral P1-2 in the right diagrams in Fig. 2, it was confirmed that when periodic acid (sodium periodate) was added, as a result of periodic acid (sodium periodate) binding to and oxidizing 4β,25(OH)₂D₃ having a 1,2-diol structure, 4β,25(OPH)₂D₃ was removed and not extracted.

Fig. 3 shows SRM chromatograms about 4β,25(OH)₂D₃ being able to be removed by the addition of periodic acid (sodium periodate).

More specifically, the left diagrams in Fig. 3 show the SRM chromatograms for columns with diatomaceous earth alone. Then, in the left diagrams in Fig. 3, the upper figure (m/z: 635.3 > 357.0) shows the SRM chromatogram at the addition of 4β,25-dihydroxyvitamin D₃ (the amount added is 180 pg (4.4 ng/mL)), while the lower figure (m/z: 638.3 > 357.0) shows the SRM chromatogram at the addition of 1α,25-dihydroxyvitamin D₃-¹³C₃ (the amount added is 180 pg (4.4 ng/mL)). In addition, the right diagrams in Fig. 3 show the SRM chromatograms for columns with sodium periodate (15 mass%) added to diatomaceous earth. Then, in the right diagrams in Fig. 3, the upper figure (m/z: 635.3 > 357.0) shows the SRM chromatogram at the addition of 4β,25-dihydroxyvitamin D₃ (the amount added was 180 pg (4.4 ng/mL)), while the lower figure (m/z: 638.3 > 357.0) shows the SRM chromatogram at the addition of 1α,25-dihydroxyvitamin D₃-¹³C₃ (the amount added is 180 pg (4.4 ng/mL)).

As is clear with reference to the region indicated by the reference numeral P2-1 in the left diagrams in Fig. 3, it was confirmed that when periodic acid (sodium periodate) was not added, 4β,25(OH)₂D₃ and 1α,25-dihydroxyvitamin D₃-¹³C₃ were not removed but extracted. Meanwhile, as is clear with reference to the region indicated by the reference numeral P2-2 in the right diagrams in Fig. 3, it was confirmed that when periodic acid (sodium periodate) was added, as a result of periodic acid (sodium periodate) binding to and oxidizing 4β,25(OH)₂D₃ having a 1,2-diol structure, 4β,25(OH)₂D₃ was removed and not extracted; however, because periodic acid (sodium periodate) does not bind to or oxidize 1α,25-dihydroxyvitamin D₃-¹³C₃ having a 1,3-diol structure, 1α,25-dihydroxyvitamin D₃-¹³C₃ was not removed but extracted. That is, 1α,25-dihydroxyvitamin D₃ (1α,25(OH)₂D₃) and 4β,25-dihydroxyvitamin D₃ (4β,25(OH)₂D₃) can be distinguished.

### <5-3. Example 3 >

### [Solid-Liquid Extraction of 1α,25-Dihydroxyvitamin D₃ Using Periodic Acid-Added Column (0.04 g of sodium periodate, 0.06 g of sodium periodate, 0.1 g of sodium periodate, 0.15 g of sodium periodate, and 0.2 g of sodium periodate are each added to 1 g of diatomaceous earth)]

1 mL of a sample diluent (30% acetonitrile solution) containing 0.2 mL of serum and an internal standard (1α,25-dihydroxyvitamin D₃-¹³C₃) was loaded onto the column. The column was allowed to stand for 1 minute, and 0.6 mL of hexane was added, followed by elution under pressure. The eluate was discarded. 0.6 mL of hexane/ethyl acetate (1:1) was added, followed by elution under pressure (repeated twice). The eluate was concentrated, and then a derivatization reagent DAP-PA (0.5 mg/mL, 0.1mL) was added and heated at 80°C for 15 minutes to cause derivatization. After concentration, the residue was dissolved with 50 µL of redissolution solvent (50% acetonitrile solution) and subjected to LC-MS/MS analysis.

The scheme of Example 3 is shown below as Scheme 4.

### (Scheme 4)

(1) Load 1 mL of a sample diluent (30% acetonitrile solution) containing 0.2 mL of serum and an internal standard (1α,25-dihydroxyvitamin D₃-¹³C₃) onto the column.
   ↓
(2) Allow to stand for 1 minute.
   ↓
(3) Add 0.6 mL of hexane and elute under pressure. Discard the eluate.
   ↓
(4) Add 0.6 mL of hexane/ethyl acetate (1:1) and elute under pressure (repeat twice).
   ↓
(5) Concentrate the eluate, add a derivatization reagent DAP-PA (0.5 mg/mL, 0.1mL), and heat at 80°C for 15 minutes to cause derivatization.
   ↓
(6) After concentration, dissolve with 50 µL of a redissolution solvent (50% acetonitrile solution).
   ↓
(7) LC-MS/MS analysis.

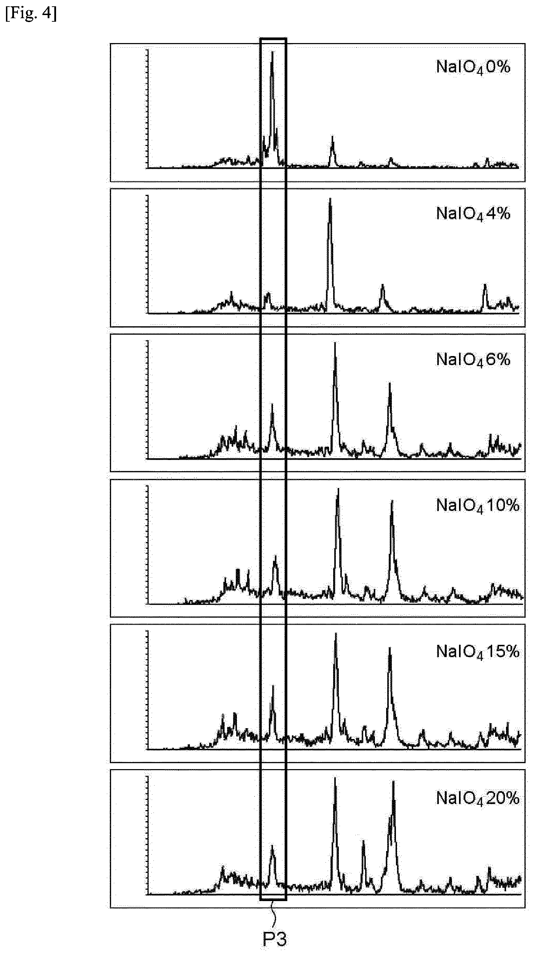

The results of the LC-MS/MS analysis of Example 3 are shown in Fig. 4. The conditions for the LC-MS/MS analysis for deriving the results of the LC-MS/MS analysis shown in Fig. 4 are shown below in Table 4.

**[Table 4]**

| Analysis Conditions | | | | | |
|---|---|---|---|---|---|
| | LC | Agilent Technologies model 1290 Infinity | | | |
| | Column | CAPCELL CORE C₁₈ (2.7 µm) 2.1 mm I.D. × 100 mm column (OSAKA SODA, Osaka, Japan) | | | |
| | Solvent | A: 0.1% Formic acid-water | | | |
| | | B: 0.1% Formic acid-acetonitrile | | | |

| | Flow rate | | 0.3 mL/min | | |
|---|---|---|---|---|---|
| | 0.01 min | 30%B | | | |
| | 0.5 | 58%B | | | |
| | 7.0 | 58%B | | | |
| | 7.01 | 90%B | | | |
| | 8.0 | 90%B | | | |
| | 8.01 | 30%B | | | |
| | 9.0 | 30%B | | | |
| | | | | | |

| | MS | | Shimadzu LCMS8040 | | |
|---|---|---|---|---|---|
| | MS/MS conditions | | *m*/*z* | CE | |
| | 1α,25(OH)₂D₃ | | 635.3 > 357.0 | 25 | |
| | 4β,25(OH)₂D₃ | | | | |
| | 1α,25(OH)₂D₃-¹³C₃ | | 638.3 > 357.0 | 25 | |

Fig. 4 shows SRM chromatograms about 4β,25(OH)₂D₃ being able to be removed by the addition of periodic acid in pooled serum.

More specifically, Fig. 4 shows, from upper to lower, the SRM chromatogram for the column with no sodium periodate added to 1 g of diatomaceous earth, the SRM chromatogram for the column with 0.04 g of sodium periodate added to 1 g of diatomaceous earth, the SRM chromatogram for the column with 0.06 g of sodium periodate added to 1 g of diatomaceous earth, the SRM chromatogram for the column with 0.1 g of sodium periodate added to 1 g of diatomaceous earth, the SRM chromatogram for the column with 0.15 g of sodium periodate added to 1 g of diatomaceous earth, and the SRM chromatogram for the column with 0.2 g of sodium periodate added to 1 g of diatomaceous earth.

As is clear with reference to the region indicated by the reference numeral P3 in Fig. 4, it was confirmed that when sodium periodate was not added, 4β,25(OH)₂D₃ was not removed but extracted (the top diagram in Fig. 4). Meanwhile, it was confirmed that when periodic acid (sodium periodate) was added (addition of 0.04 g to 0.2 g of sodium periodate to 1 g of diatomaceous earth), as a result of periodic acid (sodium periodate) binding to and oxidizing 4β,25(OH)₂D₃ having a 1,2-diol structure, 4β,25(OH)₂D₃ was removed and not extracted.

Incidentally, the present invention is not limited to the above embodiments and the above examples, and various modifications can be made without departing from the gist of the present invention.

In addition, the effects described herein are merely exemplary and not restrictive, and there may also be other effects.

In addition, the present invention may also be configured as follows.
[1]
   A solid phase mixture including:
   an oxidizing agent and/or a salt of the oxidizing agent; and
   solid phase particles.
[2]
   The solid phase mixture according to [1], wherein the oxidizing agent is a compound capable of selectively oxidizing 1,2-diol compounds.
[3]
   The solid phase mixture according to [1] or [2], wherein the oxidizing agent is periodic acid or lead tetraacetate.
[4]
   The solid phase mixture according to any one of [1] to [3], wherein the salt of the oxidizing agent is a salt of periodic acid.
[5]
   The solid phase mixture according to any one of claims 1 to 4, wherein the solid phase particles are particles of at least one kind selected from diatomaceous earth, silica gel, dextran, and a polymer.
[6]
   The solid phase mixture according to claim [5], wherein the polymer is at least one kind selected from polystyrene, polymethacrylate, and polyvinyl alcohol.
[7]
   The solid phase mixture according to any one of [1] to [6], wherein the mass of the oxidizing agent and/or the salt of the oxidizing agent relative to the mass of the solid phase particles is 4% to 20%.
[8]
   The solid phase mixture according to any one of [1] to [7], configured to selectively bind to and oxidize a 1,2-diol compound, thereby extracting a diol compound other than 1,2-diol compounds.
[9]
   The solid phase mixture according to [8], wherein
   the 1,2-diol compound is a vitamin D having a 1,2-diol structure, and
   the diol compound other than 1,2-diol compounds is a vitamin D having a diol structure other than a 1,2-diol structure.
[10]
   The solid phase mixture according to any one of [1] to [7], configured to selectively bind to and oxidize a 1,2-diol compound, thereby extracting a 1,3-diol compound.
[11]
   The solid phase mixture according to [10], wherein
   the 1,2-diol compound is a vitamin D having a 1,2-diol structure, and
   the 1,3-diol compound is a vitamin D having a 1,3-diol structure.
[12]
   The solid phase mixture according to any one of [1] to [11], wherein the oxidizing agent and/or the salt of the oxidizing agent is in a solid phase state.
[13]
   A packing material including the solid phase mixture according to any one of [1] to [12].
[14]
   A column packed with the packing material according to [13].

## Claims

1. A solid phase mixture including:
an oxidizing agent and/or a salt of the oxidizing agent; and
solid phase particles.

2. The solid phase mixture according to claim 1, wherein the oxidizing agent is a compound capable of selectively oxidizing 1,2-diol compounds.

3. The solid phase mixture according to claim 1 or 2, wherein the oxidizing agent is periodic acid or lead tetraacetate.

4. The solid phase mixture according to any one of claims 1 to 3, wherein the salt of the oxidizing agent is a salt of periodic acid.

5. The solid phase mixture according to any one of claims 1 to 4, wherein the solid phase particles are particles of at least one kind selected from diatomaceous earth, silica gel, dextran, and a polymer.

6. The solid phase mixture according to claim 5, wherein the polymer is at least one kind selected from polystyrene, polymethacrylate, and polyvinyl alcohol.

7. The solid phase mixture according to any one of claims 1 to 6, wherein the mass of the oxidizing agent and/or the salt of the oxidizing agent relative to the mass of the solid phase particles is 4% to 20%.

8. The solid phase mixture according to any one of claims 1 to 7, configured to selectively bind to and oxidize a 1,2-diol compound, thereby extracting a diol compound other than 1,2-diol compounds.

9. The solid phase mixture according to claim 8, wherein
the 1,2-diol compound is a vitamin D having a 1,2-diol structure, and
the diol compound other than 1,2-diol compounds is a vitamin D having a diol structure other than a 1,2-diol structure.

10. The solid phase mixture according to any one of claims 1 to 7, configured to selectively bind to and oxidize a 1,2-diol compound, thereby extracting a 1,3-diol compound.

11. The solid phase mixture according to claim 10, wherein
the 1,2-diol compound is a vitamin D having a 1,2-diol structure, and
the 1,3-diol compound is a vitamin D having a 1,3-diol structure.

12. The solid phase mixture according to any one of claims 1 to 11, wherein the oxidizing agent and/or the salt of the oxidizing agent is in a solid phase state.

13. A packing material including the solid phase mixture according to any one of claims 1 to 12.

14. A column packed with the packing material according to claim 13.
